# EUROPÄISCHE PATENTSCHRIFT

(11) **EP 0 600 239 B1**
(45) Veröffentlichungstag und Bekanntmachung des Hinweises auf die Patenterteilung: **15.07.1998**
(21) Anmeldenummer: 93117642.4
(22) Anmeldetag: 30.10.1993
(51) Int. Cl.: A41C 3/14, A61F 2/52

(54) **Busenkorrekturteil**
Bust correction device
Dispositif pour la correction de la poitrine

(30) Priorität: 30.10.1992 DE 9214775 U
(43) Veröffentlichungstag der Anmeldung: 08.06.1994
(73) Patentinhaber: DEKUMED Gesellschaft für Kunststoff- und Medizintechnik mbH, D-83233 Bernau (DE)
(72) Erfinder: Rickauer, Peter, D-83233 Bernau (DE)
(74) Vertreter: Patentanwälte Dipl.-Ing. R. Splanemann Dr. B. Reitzner Dipl.-Ing. K. Baronetzky

(56) Entgegenhaltungen:
- WO-A-86/01997
- DE-A- 2 605 148
- DE-C- 3 938 328
- DE-U- 9 213 880
- GB-A- 933 052
- US-A- 3 576 037

## Beschreibung

Die Erfindung betrifft ein Busen-Korrekturteil. Derartige Korrekturteile werden beispielsweise als Stützteile für von der Idealform abweichende Busen oder zur Vergrößerung von schwach entwickelten Busen oder als Ausgleichsteile nach Brustoperationen verwendet. Diese Korrekturteile bestanden bisher im allgemeinen aus Schaumstoff und wurden in die Taschen von Büstenhaltern eingenäht. Ferner sind aus den Unterlagen des deutschen Gebrauchsmusters G 9107681.1 Korrekturkörper aus einer in Kunststoffolien eingeschweißten, weich eingestellten Zweikomponenten-Siliconkautschukmasse mit einer weichen gallertartigen Konsistenz bekannt. Diese Körper können eine etwa gleiche Dicke aufweisen oder aber auch eine unterschiedliche Masseverteilung besitzen. Nähere Angaben über die spezielle Ausgestaltung dieser Körper wurden jedoch nicht gemacht.

Aus den Unterlagen des deutschen Gebrauchsmusters G 92 06 370.5 ist ein Brustkissen mit einem Körper aus einem mit einer Polyurethanfolie überzogenen additionsvernetzten Zweikomponenten-Siliconkautschuk bekannt, der eine konvexe vordere Seite und eine im wesentlichen konkave Rückseite aufweist, die beim Tragen an der Form der weiblichen Brust anliegt, wobei das Brustkissen ein Haftmittel aufweist, das mit dem Körper verbunden ist und lösbar mit der Brust verklebbar ist.

Ein derartiges Brustkissen soll u.a. bei der Teilamputationen korrigierend eingesetzt werden. Dieses Brustkissen hat bei dieser Anwendung den Nachteil, daß eine ideale Korrektur der teilamputierten Brust nicht möglich ist.

Brustoperationen werden in frühen Stadien des Brustkrebses im allgemeinen so durchgeführt, daß nicht die gesamte Brust, sondern nur die mit dem Krebsgeschwür befallenen Teile amputiert werden. Es entstehen an diesen Stellen Abflachungen oder Vertiefungen, die ausgeglichen werden müssen, damit die teilamputierte Brust wieder die Form der nichtamputierten Brust erhält.

Zur Lösung dieser Aufgabe wird ein Busen-Korrekturteil in Form eines langgestreckten, sich zu den Enden hin verjüngenden Körpers aus einer gelartig ausgehärteten Kunstharzmasse, welcher eine konvexe obere Fläche und eine untere Fläche aufweist,- wobei diese Flächen in der Draufsicht durch zwei sich schneidende Kreisbögen begrenzt sind, vorgeschlagen; dieses Korrekturteil ist dadurch gekennzeichnet, daß der Körper durch eine im wesentlichen plane oder leicht konvexe untere Fläche begrenzt ist, wobei der Körper kein Haftmittel zum lösbaren Verkleben mit der Brust aufweist.

Dieses Korrekturteil liegt mit seiner planen oder nur leicht konvexen unteren Fläche auf dem amputierten Teil der Brust auf und erzeugt mit seiner konvexen oberen Fläche eine Wölbung, die der Wölbung der nichtoperierten Brust entspricht.

Die Größe des Busenkorrekturteils kann in Abhängigkeit von der operativ entfernten Menge des Krebsgeschwürs unterschiedlich groß sein.

Vorzugsweise ist der Krümmungsradius der Kreisbögen etwa gleich.

Nach einer weiteren bevorzugten Ausführungsform ist der Krümmungsradius eines Kreisbogens im Längsschnitt etwa gleich groß und im Querschnitt etwa doppelt so groß wie der Krümmungsradius der konvexen oberen Fläche.

Weiterhin sind die Schnittpunkte der Kreisbögen vorzugsweise zu kleinen Kreisen erweitert.

Die Erfindung ist anhand der Zeichung erläutert. Es zeigen:
- Fig. 1: das Korrekturteil in der Draufsicht;
- Fig. 2: einen Längsschnitt durch das Korrekturteil von Fig. 1;
- Fig. 3: einen Querschnitt durch das Korrekturteil von Fig. 1;
- Fig. 3A: einen vergrößerten Teilquerschnitt durch den Randbereich von Fig. 3.

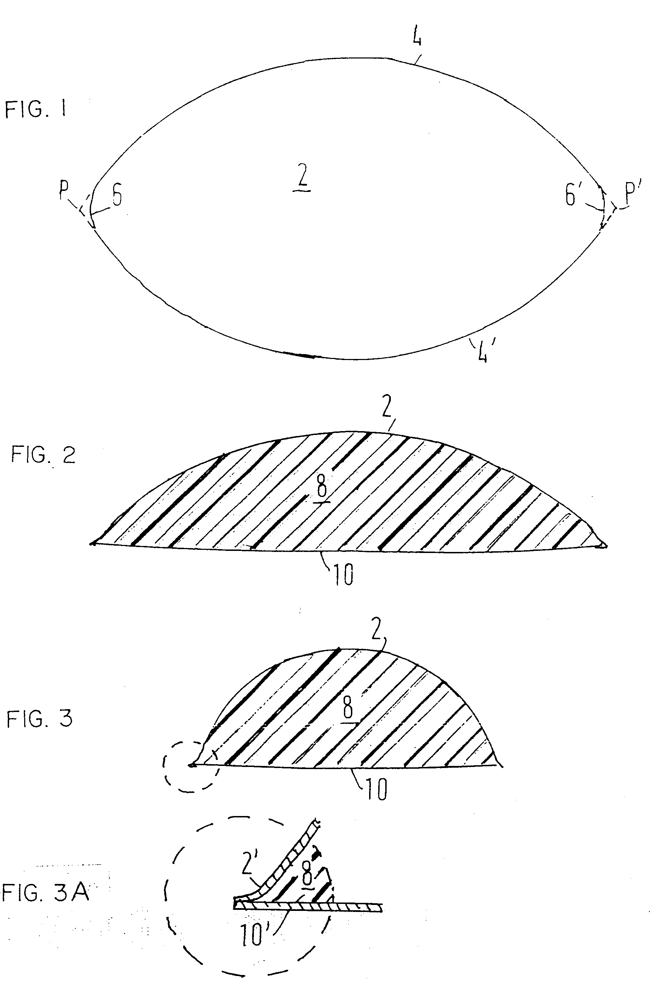

Fig. 1 zeigt in der Draufsicht die konvexe obere Fläche 2, die durch die beiden, sich schneidenden Kreisbögen 4 bzw. 4' begrenzt wird. Diese beiden Kreisbögen schneiden sich theoretisch in den Punkten P bzw. P', wie es durch die gestrichelten Linien angedeutet ist; in der Praxis sind diese Punkte aber zu kleinen Kreisen erweitert, wie es bei 6 bzw. 6' angedeutet ist.

Im Längsschnitt von Fig. 2 ist der Körper aus einer gelartig ausgehärteten Kunstharzmasse mit der Bezugszahl 8 bezeichnet. Der Körper stellt üblicherweise eine durch Additionsvernetzung oder Kondensationsvernetzung ausgehärtete Siliconharzmasse dar. Die Kunstharzmasse ist mit einer Folie ummantelt, wie es nachstehend in Verbindung mit Fig. 3A erläutert ist, da eine folienfreie Siliconharzmasse den Nachteil hat, daß sie klebrig ist, da die Siliconharzmasse häufig nicht vollständig vernetzt ist.

Die Kunstharzmasse 8 wird durch die konvexe obere Fläche 2 und die im wesentlichen plane bzw. leicht konvexe untere Fläche 10 begrenzt. Entsprechendes gilt für die Darstellung des Korrekturteils im Querschnitt gemäß Fig. 3.

Bei der dargestellten Ausführungsform sind die Krümmungsradien der Kreisbögen 4 und 4' untereinander etwa gleich. Diese Krümmungsradien sind ebenfalls gleich groß wie der Krümmungsradius der oberen Fläche 2 von Fig. 3 und etwa doppelt so groß wie der Krümmungsradius der oberen Fläche 2 von Fig. 3.

Der vergrößerte Teilquerschnitt von Fig. 3a zeigt, wie der Körper 8 aus der Kunstharzmasse zwischen zwei Folien 2' und 10' (z.B. Polyurethanfolien) eingeschweißt ist, die in diesem Fall die konvexe obere Fläche bzw. die im wesentlichen plane untere Fläche bilden.

Die Herstellung der folienummantelten Korrekturteile nach beiden Ausführungsformen kann analog dem in der DE-C-3 938 328 angegebenen Verfahren erfolgen. Hierbei wird zunächst die dem Körper abgewandte thermoplastische Folie (Bezugszahl 2' von Fig. 3A) über eine Matrize gespannt, deren Kontur der konvexen oberen Fläche 2 entspricht. Die Kunststoffolie wird durch Tiefziehen dauerhaft den Konturen der Matrize angepaßt. Die Matrize wird dann bis zu ihrem Rand mit der Kunstharzmasse 8 gefüllt. Dann wird die Kunstharzmasse mit einer zweiten Folie (Bezugszahl 10' von Fig. 3A) abgedeckt und über ihren gesamten Rand mit der ersten Folie verschweißt. Schließlich wird die Kunstharzmasse 8 ausgehärtet.

## Patentansprüche

1. Busen-Korrekturteil in Form eines langgestreckten, sich zu den Enden hin verjüngenden Körpers aus einer gelartigen ausgehärteten Kunstharzmasse, welcher eine konvexe obere Fläche (2) und eine untere Fläche (10) aufweist, wobei diese Flächen in der Draufsicht durch zwei sich schneidende Kreisbögen (4, 4') begrenzt sind, dadurch gekennzeichnet, daß der Körper (8) durch eine im wesentlichen plane oder leicht konvexe untere Fläche (10) begrenzt ist, wobei der Körper (8) kein Haftmittel zum lösbaren Verkleben mit der Brust aufweist.

2. Korrekturteil nach Anspruch 1, dadurch gekennzeichnet, daß der Krümmungsradius der Kreisbögen (4, 4') etwa gleich ist.

3. Korrekturteil nach Anspruch 1 oder 2, dadurch gekennzeichnet, daß der Krümmungsradius eines Kreisbogens (4 bzw.4') im Längsschnitt etwa gleich groß und im Querschnitt etwa doppelt so groß ist wie der Krümmungsradius der konvexen oberen Fläche (2).

4. Korrekturteil nach einem der Ansprüche 1 bis 3, dadurch gekennnzeichnet, daß die Schnittpunkte (P, P') der Kreisbögen ( 4, 4') zu kleinen Kreisen (6, 6') erweitert sind.

## Claims

1. A bust correction device in the form of an elongated body tapering off toward the ends, [said body comprised of] a gel-like, hardened plastic resine composition and having a convex upper surface (2) and a lower surface (10), said surfaces in the plan view being delimited by two circular arcs (4, 4') intercepting one another, characterized in that the body (8) is delimited by a substantially planar or slightly convex lower surface (10), wherein said body (8) has no adhesive agent for detachable adhesion to the breast.

2. A correction device according to claim 1, characterized in that the radius of curvature of said circular arcs (4, 4') is approximately identical.

3. A correction device according to claims 1 or 2, characterized in that the radius of curvature of one of said circular arcs (4 or 4') is approximately as great as said convex upper surface (2) in longitudinal section and approximately twice as great as the radius of curvature of said convex upper surface (2) in cross-section.

4. A correction device according to any one of claims 1 to 3, characterized in that the points of interception (P, P') of said circular arcs (4, 4') are enlarged to small circles (6, 6')

## Revendications

1. Dispositif pour la correction de la poitrine, en forme d'un corps allongé se rétrécissant vers ses extrémités et se composant d'une masse comparable à un gel, durcie, en résine artificielle, ledit corps ayant une surface supérieure (2) convexe et une surface inférieure (10), ces surfaces étant délimitées en vue d'en haut par deux arcs de cercle (4, 4') qui se coupent, caractérisé en ce que ledit corps (8) est délimité par une surface inférieure (10) étant essentiellement plane ou légèrement convexe, ledit corps (8) n'ayant aucun agent d'adhésion pour le collage détachable à la poitrine.

2. Dispositif de correction selon la revendication 1, caractérisé en ce que les rayons de courbure desdits arcs de cercle (4, 4') sont à peu près identiques.

3. Dispositif de correction selon l'une des revendications 1 et 2, caractérisé en ce que le rayon de courbure de l'un desdits arcs de cercle (4 or 4'), en coupe longitudinale, est à peu près de la même grandeur, et, en coupe transversale, est à peu près de la double grandeur du rayon de courbure de la surface supérieure (2) convexe.

4. Dispositif de correction selon l'une des revendications 1 à 3, caractérisé en ce que les points d'intersection (P, P') desdits arcs de cercle (4, 4') sont élargis à de petits cercles (6, 6').
